# EUROPEAN PATENT APPLICATION

(11) **EP 4 374 805 A1**
(43) Date of publication of application: **29.05.2024**
(21) Application number: 22844896.5
(22) Date of filing: 15.04.2022
(51) Int. Cl.: A61B 17/34

(54) **SEALING DEVICE AND PUNCTURE DEVICE**

(30) Priority: 17.07.2021 CN 202110809859
(71) Applicant: Shenzhen Edge Medical Co., Ltd., Shenzhen, Guangdong 518000 (CN)
(72) Inventor: LIU, Fang, Shenzhen, Guangdong 518000 (CN)
(74) Representative: Biallo, Dario
(86) International application number: PCT/CN2022/087099
(87) International publication number: WO 2023/000724

(57) **Abstract**

Provided in the present application are a sealing device and a puncture device. The sealing device includes: a first body; a second body, the second body being connected to the first body, and the second body and the first body forming an instrument channel for an instrument to pass therethrough; and a sealing component, the sealing portion being arranged in the instrument channel and allowing the instrument to pass there through, so as to form at least one sealing part in the instrument channel in a state in which the instrument is inserted and/or not inserted into the instrument channel. By means of the implementation, the sealing device can form at least one sealing part in the instrument channel in the state in which the instrument is inserted or not inserted into the instrument channel, such that the sealing performance of the puncture device can be significantly improved.

## Description

### CROSS-REFERENCE TO RELATED APPLICATIONS

This application claims priority to Chinese Patent Application No. CN 202110809859.7, entitled "Sealing Device and Puncture Device", and filed on July 17, 2021, the contents of which are incorporated herein by reference.

### TECHNICAL FIELD

The present application relates to the field of medical instruments, and in particular to a sealing device and a puncture device with the sealing device.

### BACKGROUND

The puncture device is a common tool for assisting surgery. During surgery, gas is inflated into the surgical area through the incision on the patient's body, so that the surgical space is expanded to facilitate surgery, after the gas is inflated, the puncture device is inserted into the surgical space, and the surgical space of the abdominal cavity is sealed through the sealing structure of the puncture device. During surgery, the surgical instrument enters the surgical space through the puncture device, and the surgical space is continuously sealed through the sealing structure of the puncture device.

At present, due to the fact that the puncture device is still poor in sealing performance in the process of insertion and removal of the surgical instrument, the puncture device needs to be continuously improved.

### SUMMARY

Based on this, it is necessary to provide a sealing device and a puncture device with strong sealing performance.

In order to solve the above technical problem, the present application provides a sealing device, including: a first body; a second body, in which, the second body is connected to the first body to form a through instrument channel for an instrument to pass through; and a sealing portion, in which, the sealing portion is arranged in the instrument channel, and configured to allow the instrument to pass through, so as to form at least one sealing position in the instrument channel when the instrument is inserted and/or is not inserted into the instrument channel.

In an embodiment, the sealing portion includes a sealing plate, the sealing plate is provided with a sealing opening, the sealing opening is aligned with the instrument channel, the sealing opening is configured for allowing the instrument to pass through, and in a state that the instrument is inserted into the instrument channel, an inner periphery of the sealing opening abuts against the instrument to form a sealing position.

In an embodiment, the sealing portion includes a sealing door, the instrument channel includes an instrument outlet, the instrument outlet is disposed on the second body, and the sealing door is rotatably connected to the second body to seal the instrument outlet in an openable manner.

In an embodiment, the sealing portion includes an elastic element, the elastic element is connected to the second body and the sealing door, and the elastic element provides an elastic force for pressing the sealing door on the instrument outlet to achieve sealing.

In an embodiment, the elastic element includes at least one of a torsion spring, a tension spring, and a leaf spring.

In an embodiment, an included angle between a plane where the instrument outlet is located and a central axis of the first body and/or the second body is 15°-45°.

In an embodiment, the sealing door has an opening and closing angle of 15°-45°.

In an embodiment, the sealing door includes a rotating shaft, and a height between the sealing plate and the rotating shaft is related to a cross-sectional area of the instrument channel located between the sealing plate and the instrument outlet.

In an embodiment, the height is negatively correlated with the cross-sectional area.

In an embodiment, the height is between 0 mm and 46 mm.

In an embodiment, the height is between 0 mm and 21 mm.

In an embodiment, a protrusion is provided on one side of the sealing door that seals the instrument outlet, and the protrusion is accommodated in the instrument channel when the sealing door is closed in the instrument outlet.

In an embodiment, the protrusion has an arc chamfer.

In an embodiment, a mounting slot is formed along a periphery of a base of the protrusion, the mounting slot is sleeved with a sealing ring, and the sealing ring is tightly fitted to the instrument outlet to seal the instrument outlet when the sealing door is closed in the instrument outlet, in which, the sealing ring covers a gap between the sealing door and the instrument outlet.

In an embodiment, the sealing door has a first magnetic portion, the instrument outlet has a second magnetic portion that attracts the first magnetic portion, and the sealing position is sealed between the sealing door and the instrument outlet through a magnetic connection between the first magnetic portion and the second magnetic portion.

In an embodiment, the first body is provided with a first channel, the instrument channel includes the first channel, the first channel includes a first sub-channel and a second sub-channel that are interconnected, the first sub-channel includes an instrument inlet and a first middle hole, the second sub-channel includes the first middle hole and the second middle hole, the instrument inlet, the first middle hole and the second middle hole are successively connected, and a size of the first middle hole is smaller than a size of the instrument inlet.

In an embodiment, the first sub-channel gradually shrinks from the instrument inlet to the first middle hole; and/or the second sub-channel gradually expands from the first middle hole to the second middle hole.

In an embodiment, the second body is provided with a second channel, the instrument channel includes the second channel, the second channel includes a third middle hole and an instrument outlet, and the instrument inlet, the first middle hole, the second middle hole, the third middle hole and the instrument outlet are connected in sequence.

In an embodiment, the sealing opening includes a circular opening; and the size of the sealing opening is smaller than or equal to the size of the insertion portion of the instrument.

In an embodiment, the sealing plate includes a first portion, the first portion includes a middle region and an edge region disposed around the middle region, the sealing opening is disposed in the middle region, the middle region includes a transitional region, the transitional region includes a region extending from a periphery of the sealing opening to an inner periphery of the edge region, and a thickness of the transitional region is smaller than a thickness of the edge region.

In an embodiment, the thickness of the transitional region increases gradually from the periphery of the sealing opening to the inner periphery of the edge region.

In an embodiment, the middle region is fan-shaped.

In an embodiment, the sealing plate is arranged at an engaged position of the first body and the second body.

In an embodiment, at the engaged position, the first body is provided with a first boss, the second body is provided with a second boss, the structures of the first boss and the second boss are matched with the structure of the edge region, the second boss supports the edge region, and the first boss tightly compacts the edge region on the second boss.

In an embodiment, the second boss is recessed to form a groove, a structure of the groove matches a structure of the transitional region, the first boss compacts the edge region tightly to the second boss, and the transitional region is aligned with the groove.

In an embodiment, the first boss is provided with a mounting rib, the sealing plate is provided with a mounting groove matching the mounting rib, and the sealing plate is limited by wedging between the mounting groove and the mounting rib when the sealing plate is mounted on the second body.

In an embodiment, the instrument channel is one of four instrument channels, the sealing opening is one of four sealing openings, and different sealing openings are aligned with different instrument channels, respectively.

In an embodiment, the sealing plate is disposed at an engaged position of the first body and the second body, the first boss is provided with two orthogonal mounting ribs, the sealing plate is provided with two orthogonal mounting grooves matching the mounting ribs, and the sealing plate is limited by wedging between the orthogonal mounting grooves and the orthogonal mounting ribs when the sealing plate is mounted on the second body.

In an embodiment, the four instrument channels include two first instrument channels with a first size and two second instrument channels with a second size, the first size is greater than the second size, the two first instrument channels are arranged diagonally, the two second instrument channels are arranged diagonally, and the two first instrument channels and the two second instrument channels are orthogonally arranged.

In an embodiment, the second body is provided with a first fool-proof structure at the engaged position, a periphery of the sealing plate is provided with a second fool-proof structure, the first body is provided with a third fool-proof structure at the engaged position, and the second fool-proof structure and the third fool-proof structure are matched with the first fool-proof structure

In an embodiment, the sealing portion includes a sealing plate, the sealing plate has a sealing opening with a diameter smaller than that of the instrument, the sealing opening is aligned with the instrument channel, and an edge of the sealing opening extends towards a center of the sealing opening to form a plurality of openable flaps;
in a state in which the instrument is inserted into the sealing opening, the plurality of flaps are opened; or

In a state in which the instrument is not inserted into the sealing opening, the plurality of flaps are closed to form the sealing portion.

In an embodiment, the plurality of flaps include flaps having a high coefficient of elasticity.

In an embodiment, the plurality of flaps include fan-shaped flaps having equal or unequal arc angles, and the circle centers of the plurality of flaps include a circle center of the sealing opening.

In an embodiment, the sealing portion includes at least two sealing plates, and the at least two sealing plates are stacked.

In an embodiment, the plurality of flaps of adjacent sealing plates are interleaved.

In an embodiment, the sealing portion includes a sealing plate made of a porous elastic material of high polymer, and/or the sealing portion includes a sealing plate made of an aerogel material;
In a state where the instrument is inserted into the sealing plate, the sealing plate deforms in the insertion area of the instrument to generate a sealing opening for the instrument to pass through, and the sealing opening abuts against the instrument to form the sealing position in the state that the instrument is inserted into the sealing plate; and/or, in a state where the instrument is not inserted into the sealing plate, the sealing plate remains flat or returns to flat to form the sealing position.

In an embodiment, the first body and the second body are integrally formed.

In an embodiment, the outer periphery of the second body protrudes outwards to form a block, and the block is configured to connect with a slot of a guiding cannula to realize a connection between the second body and the guiding cannula.

In an embodiment, a mounting recess is formed in the outer periphery of the second body, the mounting recess is sleeved with an annular ring seal, and the annular ring seal is configured to achieve a seal between the second body and the guiding cannula when the annular ring seal is connected with the guiding cannula.

In order to solve the above technical problem, the present application further provides a puncture device, the puncture device including a guiding cannula and a sealing device as described in any one of the above embodiments installed on the guiding cannula.

In an embodiment, the instrument channel in the sealing device includes a first instrument channel for a first instrument to pass through, the guiding cannula includes a connecting insert configured for connecting with a slave operating device, and in the state where the sealing device is installed in the guiding cannula, the orientation of the first instrument channel is the same as that of the connecting insert.

In an embodiment, the first instrument includes a surgical instrument with a distal end portion being an endoscope.

In an embodiment, the outer periphery of the sealing device is provided with a block, and the inner periphery of the guiding cannula is provided with a notch and a slot that communicate with each other, the slot is configured to accommodate the block inserted from the notch, and in the state that the second body is connected with the guiding cannula, the block is accommodated in the slot, and the block is staggered with the notch.

In an embodiment, a limiting structure is arranged in the slot, and the block can rotate in the slot and is installed under the limit of the limiting structure.

In an embodiment, the block includes a first block with a first size and a second block with a second size, the notch includes a first notch with a third size and a second notch with a fourth size, the first size is smaller than the second size, the third size is greater than the first size and smaller than the second size, the fourth size is greater than the second size, and the block is allowed to be inserted into the slot under the condition that the first block is aligned with the first notch and the second block is aligned with the second notch.

The sealing device and the puncture device of the present application have the following beneficial effects:

By providing the sealing portion in the instrument channel that allows the instrument to pass through, and forming at least one sealing position in the instrument channel with or without the instrument insertion, the sealing performance of the puncture device can be remarkably improved, and the reliable use of the puncture device can be ensured.

### BRIEF DESCRIPTION OF THE DRAWINGS

FIG. 1 is a schematic structural view of a surgical robot according to an embodiment of the present application;
FIG. 2 is a partial schematic diagram of an embodiment of a surgical robot according to the present application;
FIG. 3 is a partial schematic diagram of an embodiment of a surgical instrument in a surgical robot of the present application;
FIG. 4 is a schematic diagram of structure and use of an embodiment of a puncture device of the present application;
FIG. 5 is an exploded view of an embodiment of the puncture device shown in FIG. 4;
FIG. 6 is an exploded view of an embodiment of a sealing device in the puncture device shown in FIG. 5;
FIG. 7 is a longitudinal cross-sectional view of an embodiment of the sealing device shown in FIG. 6;
FIG. 8 is a schematic diagram of an embodiment of a sealing door in the sealing device shown in FIG. 6;
FIG. 9 is a schematic diagram of an embodiment of a sealing plate in the sealing device shown in FIG. 6;
FIG. 10 is a schematic bottom view of an embodiment of the sealing plate shown in FIG. 9;
FIG. 11 is a cross-sectional view taken along the D-D direction shown in FIG. 9;
FIG. 12 is a schematic top view of an embodiment of the sealing device shown in FIG. 6;
FIG. 13 is a schematic top view of an embodiment of a second body of the sealing device shown in FIG. 6;
FIG. 14 is a schematic bottom view of an embodiment of the first body of the sealing device shown in FIG. 6; and
FIG. 15 is a schematic diagram of a sealing plate according to another embodiment of the present application.

### DETAILED DESCRIPTION

For ease of understanding of the present disclosure, the present disclosure will be described more fully hereinafter with reference to the associated drawings. Some embodiments of the present disclosure are set forth in the accompanying drawings. This application may, however, be embodied in many different forms and is not limited to the embodiments described herein. Rather, these embodiments are provided for the purpose of providing a more thorough and complete understanding of the disclosure of the present disclosure.

It should be noted that when an element is referred to as being "disposed on" another element, it can be directly on the other element or intervening elements may also be present. When an element is referred to as being "connected" to another element, it can be directly connected to the other element or intervening elements may also be present. When an element is considered to be "coupled" to another element, it can be directly coupled to the other element or intervening elements may also be present. The terms "vertical," "horizontal," "left," "right," and similar expressions used herein are for illustrative purposes only and are not meant to be the only way of implementation. As used herein, the terms "distal" and "proximal" are used as terms, which are customary terms in the field of interventional medical instruments, in which, "distal" denotes an end away from an operator during surgery, and "proximal" denotes an end close to the operator during surgery.

Unless otherwise defined, all technical and scientific terms used herein have the same meaning as commonly understood by one skilled in the art. The terminology used herein is for the purpose of describing particular embodiments only and is not intended to be limiting of the present application. As used herein, the term "and/or" includes all combinations of one or more of the associated listed items.

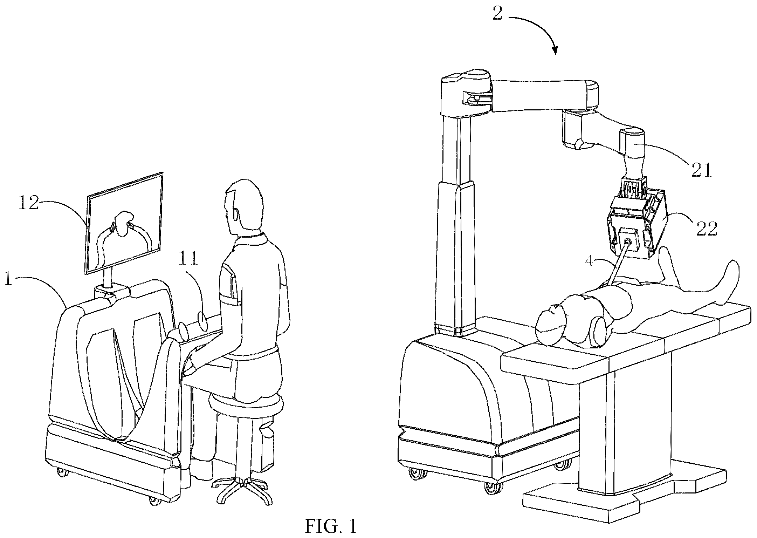

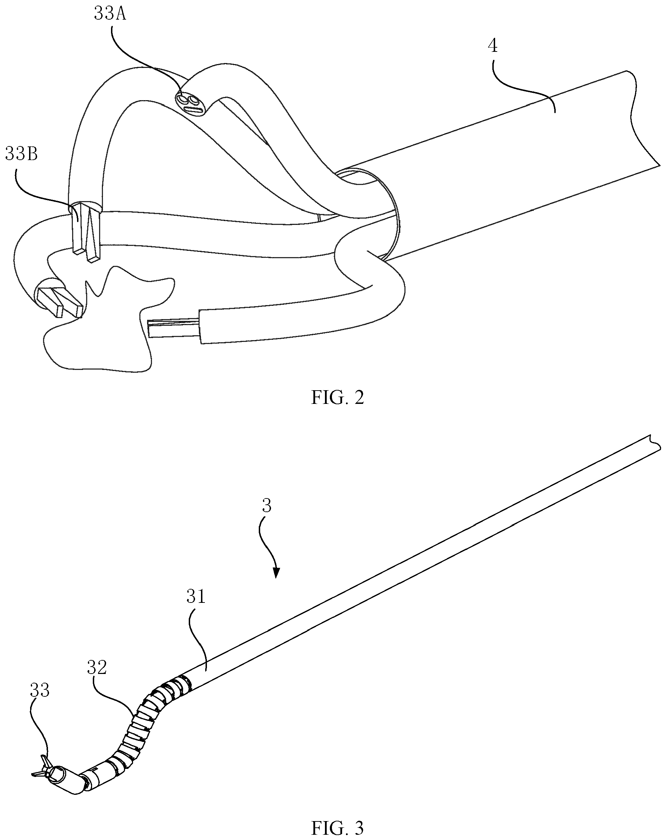

Referring to FIG. 1 to FIG. 5, the surgical robot includes a main operating console 1 and a slave operating device 2 controlled by the main operating console 1, the main operating console 1 has an operating portion 11 and a display portion 12, and a doctor sends a control command to the slave operating device 2 through the operating portion 11, so that the slave operating device 2 executes a corresponding operation according to the control command, and the doctor observes the operation area through the display portion 12.

The slave operating device 2 includes a mechanical arm 21, a power mechanism 22 disposed at a distal end of the mechanical arm 21, and a surgical instrument 3 detachably mounted on the power mechanism 22. The power mechanism 22 is further detachably provided with a puncture device 4, which is configured to penetrate the incision on the patient's body to close the operation space S inside the incision, so as to avoid the operation from being affected by air leakage in the operation space S. The surgical instrument 3 is enabled to pass through the puncture device 4 to reach the surgical space S, and be driven by the power mechanism 22 to perform an operation.

The surgical instrument 3 includes a connecting rod 31, a connecting assembly 32 and an terminal instrument 33 sequentially connected from a proximal end to a distal end, and the connecting assembly 32 includes a plurality of joint assemblies (not labeled). The terminal instrument 33 includes an image terminal instrument 33a for collecting images within the field of view, for example, an endoscope, and an operating terminal instrument 33b for performing a surgical operation such as cutting and suturing, for example, an electrosurgical tools, a grip, an ultrasonic scalpel.

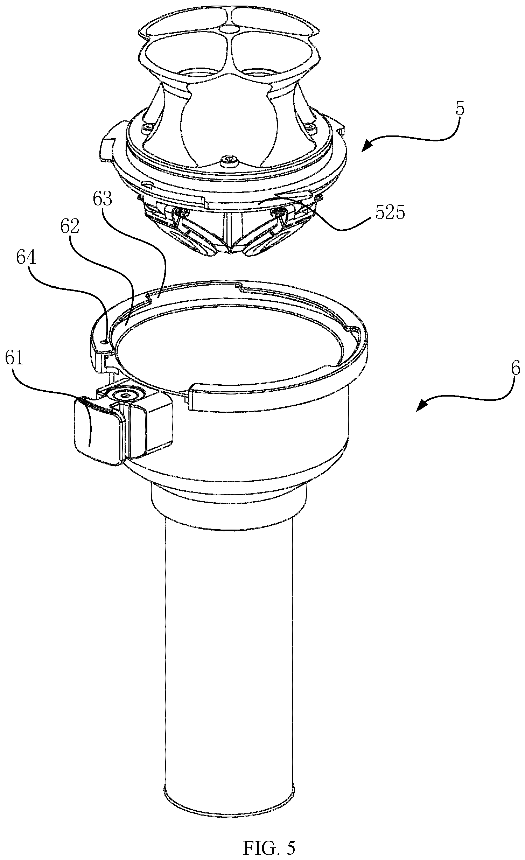

In an embodiment, as shown in FIG. 5, the puncture device 4 includes a sealing device 5 and a guiding cannula 6, the guiding cannula 6 is connected to the sealing device, the guiding cannula 6 includes a first connecting insert 61, the power mechanism 22 includes a second connector (not shown), and the first connecting insert 61 and the second connector are matched and form an insertion connection therebetween to achieve the installation of the guiding cannula 6 on the power mechanism 22, thereby achieving the installation of the puncture device 4 on the power mechanism 22.

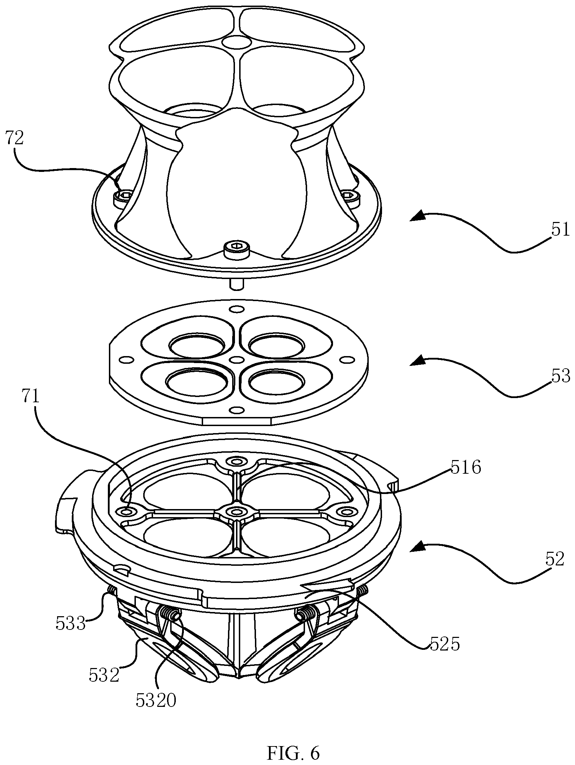

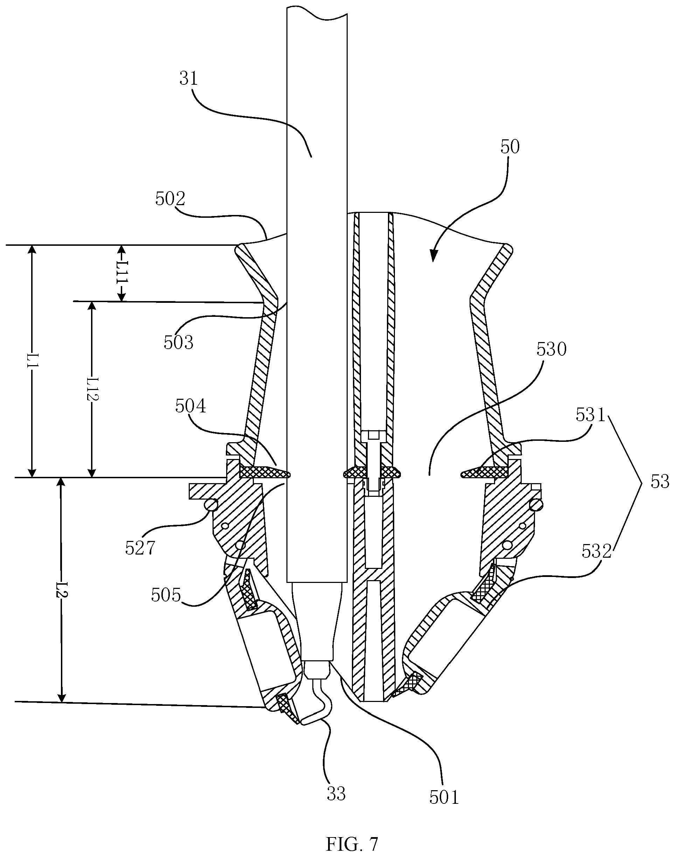

Referring to FIGS. 6 and 7, the sealing device 5 includes a first body 51 and a second body 52, and the first body 51 and the second body 52 are connected. The first body 51 and the second body 52 form a through instrument channel 50 for allowing the instrument to pass through, allowing the instrument to be inserted or pulled out of the instrument channel 50. The instrument described in this application may include a surgical instrument 3, and may also include other non-surgical instruments.

The sealing device 5 further includes a sealing portion 53 disposed at the instrument channel 50, and the instrument can pass through the sealing portion 53. The sealing portion 53 is configured to form at least one sealing position in the instrument channel 50 in the presence or in the absence of insertion of the instrument, so as to improve the overall sealing performance of the sealing device 5.

In an embodiment, the sealing portion 53 includes at least one sealing plate 531, the sealing plate 531 is made of a flexible material. Preferably, the sealing plate 531 is made of an elastic material such as silicone gel. The sealing plate 531 is provided with a sealing opening 530 aligned with the instrument channel 50 to allow the instrument to pass through the instrument channel 50 and the sealing opening 530 in the sealing plate 531. In an embodiment, the number of sealing plates 531 may further be more than two. In the case of including more than two sealing plates 531, one of the sealing openings 530 in the individual sealing plate 531 is correspondingly aligned with one instrument channel 50, and the sealing openings 530 in the adjacent sealing plates 531 are correspondingly aligned to allow the instrument to pass through the instrument channel 50 and the sealing openings 530 in the different sealing plates 531.

The "alignment" that may be referred to herein between the opening and the channel, between the opening and the opening, and/or between the channel and the channel, may involve both complete alignment and substantial alignment. Taking that the alignment is between the opening and the channel, the sealing opening serves as a hole and the instrument channel serves as a channel as an example, "complete alignment" includes the condition that the sealing opening and the instrument channel are not staggered so as to ensure that the instrument can pass through the sealing opening and the instrument channel; and the " substantial alignment" includes situations where the sealing opening and the instrument channel are staggered, but still can ensure that the instrument can pass through the sealing opening and the instrument channel.

Further, continue referring to FIG. 6 and FIG. 7, the sealing portion 53 includes a sealing door 532, the instrument channel 50 includes an instrument outlet 501 for the instrument to extend out, during operation, the instrument is inserted into the surgical space S from the instrument outlet 501. The instrument outlet 501 is disposed on the second body 52, and the sealing plate 53 is rotatably connected to the second body 52 to releasably seal the instrument outlet 501.

The instrument channel 50 includes two states, respectively a first state and a second state, specifically:
The first state includes a state in which an instrument is inserted into the instrument channel 50. For example, the first state includes a state of the instrument passing through the sealing opening 530 of the sealing plate 531, which may occur during an insertion process and/or a pull-out process of the instrument. For example, in the insertion process of the instrument, the first state includes the state of the instrument fully inserted into the instrument channel 50 (e.g. the distal portion of the instrument (e.g. the terminal instrument 33 of the surgical instrument 3) extends from the instrument outlet 501 of the instrument channel 50), and/or the distal portion of the instrument is inserted into the sealing opening 530 of the sealing plate 531 and does not extend from the instrument outlet 501 of the instrument channel 50. As another example, in the pull-out process of the instrument, the first state includes that the distal portion of the instrument is not retracted from the instrument outlet 501 of the instrument channel 50, and/or the distal portion of the instrument is withdrawn from the instrument outlet 501 of the instrument channel 50 and is not withdrawn from the sealing opening 530 of the sealing plate 531.

The second state includes a state in which no instrument is inserted into the instrument channel 50. For example, the second state includes a state in which the instrument does not pass through the sealing opening 530 of the sealing plate 531, which may occur during an insertion process and/or a pull-out process of the instrument. For example, in the insertion process of the instrument, the second state includes the state in which the instrument is not inserted into the instrument channel 50 at all (e.g. the distal portion of the instrument is not inserted into the instrument inlet 502 of the instrument channel 50), and/or the distal portion of the instrument is inserted into the instrument channel 50 and does not pass through the sealing opening 530 of the sealing plate 531. For another example, in the pull-out process of the instrument, the second state includes that the instrument is completely pulled out of the instrument channel 50, and/or the distal portion of the instrument is withdrawn from the sealing opening 530 of the sealing plate 531 and is still located in the instrument channel 50.

Regardless of whether the instrument channel 50 is in the first state or the second state, the sealing device 5 of the present application can form at least one sealing position on the instrument channel 50 through the provided sealing portion 53.

Exemplarily, in the first state, for example, in a state in which the instrument is completely inserted into the instrument channel 50, since the sealing door 532 is opened under the abutting action of the instrument, a sealing position cannot be formed at the instrument outlet 501, however, since the inner periphery of the sealing opening 530 tightly abuts against the instrument (such as the connecting rod 31 of the surgical instrument 3), a sealing position can be formed at the sealing opening 530. For another example, when the distal portion of the instrument is inserted into the sealing opening 530 of the sealing plate 531 and does not extend from the instrument outlet 501 of the instrument channel 50, the sealing door 532 closed at the instrument outlet 501 without being pushed by the instrument, so that the first sealing portion can be formed at the instrument outlet 501, and the second sealing portion can be formed at the sealing opening 530 at the same time due to the tight abutting of the inner periphery of the sealing opening 530.

For example, in the second state, for example, in a state where the instrument is completely pulled out of the instrument channel 50, since the sealing door 532 is closed at the instrument outlet 501 without being pushed by the instrument, a sealing position can be formed at the instrument outlet 501.

In one embodiment, referring to FIG. 6, the sealing portion 53 includes an elastic member 533 connecting the second body 52 and the sealing door, and the elastic member 533 provides an elastic force for pressing the sealing door 532 on the instrument outlet 501 to achieve sealing. With the arrangement of the elastic element 533, when the distal portion of the instrument does not extend out of the instrument outlet 501 and/or the distal portion of the instrument is retracted from the instrument outlet 501, the sealing door 532 can be automatically closed to the instrument outlet 501. Exemplarily, the elastic element 533 includes at least one of a torsion spring, a tension spring and a leaf spring.

In an embodiment, as shown in FIG. 6, an included angle of 15° -45° is formed between the plane where the instrument outlet 501 is located and a central axis of the first body 51 and/or the second body 52, for example, the included angle is 15°, 30° or 45°. The sealing door 532 has an opening and closing angle of 15° -45°, for example, the opening and closing angle is 15°, 30° or 45°. The opening and closing angle includes the angle of the sealing door 532 opened when the instrument extends out of the instrument outlet 501 and the instrument abuts against the sealing door 532. The smaller the included angle, the smaller the opening and closing angle generated by the instrument abutting the sealing door 532, which can effectively protect the restoring force of the elastic element 533 due to the fact that the opening and closing angle of the sealing door 532 is small, so that the service life of the elastic element 533 is prolonged, and the sealing performance is maintained. Moreover the instrument does not need to overcome too much the resistance generated by the elastic element 533 when extending out of the instrument outlet 501, so that the instrument extends out of the instrument outlet 501 more easily and smoothly. Since the resistance to be overcome for inserting the instrument is reduced, on one hand, for a manual insertion instrument, the doctor can use less force, more comfortable hand feel to achieve the insertion of the instrument, and on the other hand, for a motor-driven insertion instrument, it helps to protect the motor and reduce power consumption.

In an embodiment, referring to FIG. 6, the sealing door 532 includes a rotating shaft 5320, and the sealing door 532 is rotatably connected to the second body 52 by using the rotating shaft 5320 and is opened and closed by rotation. A height between the sealing plate 531 and the rotating shaft 5320 can be determined and designed according to a cross-sectional area of a section of the instrument channel 50 located between the sealing plate 531 and the instrument outlet 501. Preferably, the height is negatively correlated with the area, that is, if the area is larger, the height can be designed to be less. Therefore, it can be designed at a suitable height to reduce the negative pressure pulling force caused by the negative pressure in the section of the instrument channel 50 between the sealing plate 531 and the instrument outlet 501 when the instrument is pulled out, on one hand, for a manual pulling-out instrument, the doctor can easily pull out the instrument with a small force and a more comfortable hand feeling, and on the other hand, for a motor-driven pulling-out instrument, it helps to protect the motor and reduce power consumption.

Exemplarily, the height may be designed to be between 0 mm and 46 mm, so that a doctor can pull out the instrument with a force of 0-4 N. More preferably, the height can be designed between 0 mm and 21 mm, so that the doctor can pull out the instrument with a force of 2-3 N. Such a design can bring good user experience for a manual pulling-out instrument.

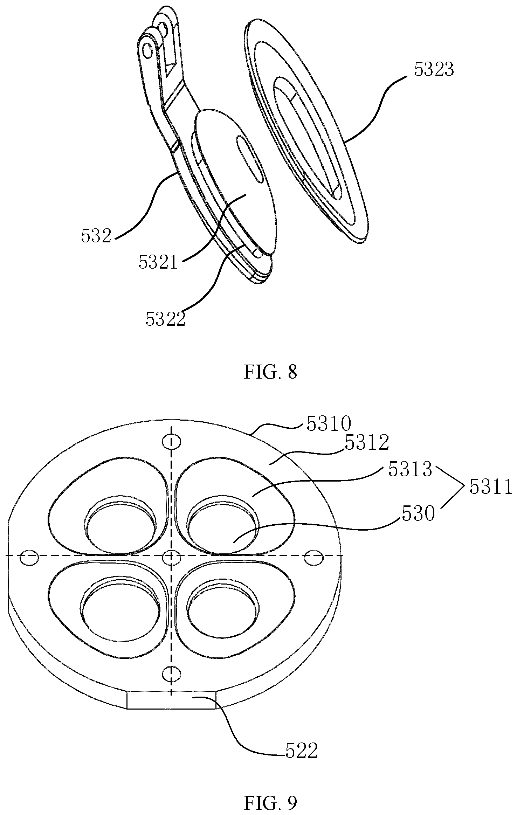

In an embodiment, as shown in FIG. 8, one side of the sealing door 532 sealing the instrument outlet 501 is provided with a protrusion 5321, and the protrusion 5321 is accommodated in the instrument channel 50 when the sealing door 532 is closed in the instrument outlet 501. With the design of the protrusion 5321, on one hand, the opening and closing angle of the sealing door 532 can be small, and on the other hand, the distal portion of the instrument contacts the protrusion 5321 in the form of a point-plane contact, so that the instrument can be easily extended from the instrument outlet 501. Preferably, the protrusion 5321 has an arc chamfer, thereby facilitating the instrument to more easily extend from the instrument outlet 501.

Preferably, a mounting slot 5322 is formed at a base of the protrusion 5321 along a periphery of the protrusion 5321, the mounting slot 5322 is sleeved with a sealing ring 5323, the sealing ring 5323 is made of a flexible material, and preferably, the sealing ring 5323 is made of an elastic material such as silicone gel. When the sealing door 532 is closed in the instrument outlet 501, the sealing ring 5323 closely fits the instrument outlet 501 to seal the instrument outlet 501, and specifically, the sealing ring 5323 covers a gap between the sealing door 532 and the instrument outlet 501 to achieve better sealing. On the one hand, the sealing ring 5323 is easy to install and is not easy to fall off, and on the other hand, since the sealing ring 5323 is arranged at the base of the protrusion 5321, the instrument abuts against the protrusion 5321 instead of the sealing ring 5323 when extending out of the instrument channel 50, so that the damage to the structure of the sealing ring 5323 can be prevented, and the sealing performance can be maintained.

In one embodiment, the sealing door 532 has a first magnetic portion, which can replace the elastic element 533 of the sealing door 532, or combine with the elastic element 533, the instrument outlet 501 has a second magnetic portion, the first magnetic portion and the second magnetic portion attract each other, and a sealing position is sealed between the sealing door 532 and the instrument outlet 501 through the magnetic connection between the first magnetic portion and the second magnetic portion. For example, the two magnetic portions are permanent magnets, and for another example, one of the two magnetic portions is a permanent magnet, and the other magnetic portion is a component made of a magnetic material, and the magnetic material includes but is not limited to materials such as iron and iron-nickel alloy.

In an embodiment, referring to FIG. 7, the first body 51 is provided with a first channel L1, the instrument channel 50 includes the first channel L1, further, the first channel L1 includes a first sub-channel L11 and a second sub-channel L12 that are interconnected, the first sub-channel L11 includes an instrument inlet 502 and a first middle hole 503, the second sub-channel L12 includes a first middle hole 503 and a second middle hole 504, that is, the first middle hole 503 belongs to a common part of the first sub-channel L11 and the second sub-channel L12. The instrument inlet 502, the first middle hole 503 and the second middle hole 504 are connected in sequence, for example, when the instrument channel 50 extends along a straight line, the first sub-channel L11 and the second sub-channel L12 are aligned, that is, the instrument inlet 502, the first middle hole 503 and the second middle hole 504 are aligned in sequence.

Preferably, the size of the first middle hole 503 is smaller than the size of the instrument inlet 502, thereby guiding the insertion operation of the instrument by the large size of the instrument inlet 502 and the small size of the first middle hole 503, and limiting the shaking of the instrument, such as the connecting rod 31 of the surgical instrument 3, in the circumferential direction of the first middle hole 503 by using the first middle hole 503 after the instrument passes through the first middle hole 503, and thus help to maintain the sealing performance of the instrument channel 50.

Preferably, the first sub-channel L11 gradually shrinks from the instrument inlet 502 to the first middle hole 503. In this way, the guiding effect on the instrument is better.

Preferably, the second sub-channel L12 gradually expands from the first middle hole 503 to the second middle hole 504. In such a structural design, a large space can be provided in the second sub-channel L12, and on one hand, even if the sealing sleeve (not labeled) sleeved by the connecting assembly 32 of the instrument, such as the surgical instrument 3, is accumulated in the second sub-channel L12, the normal movement of the instrument will not be restricted due to the small channel space, and on the other hand, in the case where the sealing plate 531 is provided at the engaged position between the first body 51 and the second body 52 as described later, a large deformation space can be provided for the sealing plate 531 to improve the sealing performance.

The second body 52 is provided with a second channel L2, the instrument channel 50 includes the second channel L2, and the second channel L2 includes a third middle hole 505 and an instrument outlet 501, and the instrument inlet 502, the first middle hole 503, the second middle hole 504, the third middle hole 505 and the instrument outlet 501 are connected sequentially. Exemplarily, when the instrument channel 50 extends along a straight line, the first channel L1 and the second channel L2 are aligned, that is, the instrument inlet 502, the first middle hole 503, the second middle hole 504, the third middle hole 505 and the instrument outlet 501 are aligned sequentially.

In one embodiment, the size of the sealing opening 530 is smaller than or equal to the size of the insertion portion of the instrument. When the surgical instrument 3 is inserted into the instrument channel 50 for normal use, the insertion portion in close contact with the sealing opening 530 is generally the connecting rod 31 of the surgical instrument 3, and thus, in one embodiment, the sealing opening 530 may include a circular opening, the size of the sealing opening 530 includes the diameter of the sealing opening 530, and the size of the insertion portion of the instrument includes the diameter of the connecting rod 31. In such a structural design, the abutting between the sealing opening 530 and the instrument can be tighter, thereby improving the sealing performance. In particular, when the first middle hole 503 having a size smaller than the size of the instrument inlet 502 is used in combination, the shaking of the instrument in the circumferential direction of the first middle hole 503 can be effectively limited.

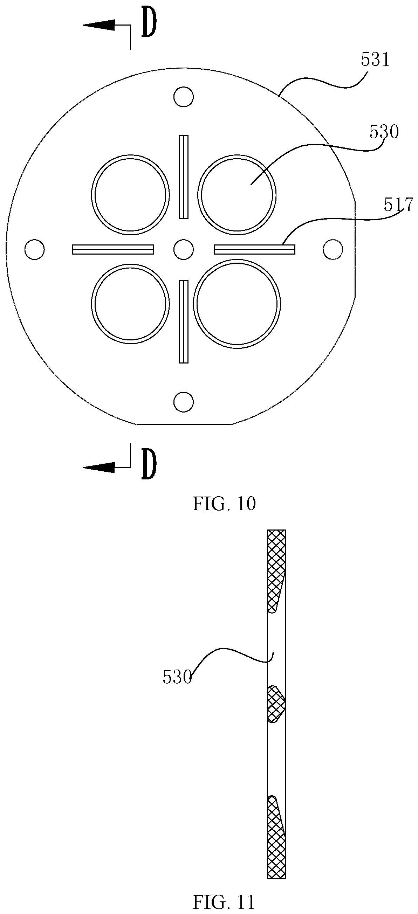

In an embodiment, referring to FIGS. 9 and 11, the sealing plate 531 includes a first portion 5310, and the number of first portions 5310 may be determined according to the number of instrument channels 50, for example, the number of first portions 5310 is equal to the number of instrument channels 50. The first portion 5310 includes a middle region 5311 and an edge region 5312 arranged around the middle region 5311, the sealing opening 530 is disposed in the middle region 5311, the middle region 5311 includes a transitional region 5313, the transitional region 5313 includes a region extending from a periphery of the sealing opening 530 to an inner edge of the edge region, and a thickness of the transitional region 5313 is smaller than a thickness of the edge region 5312. In this way, even if the instrument inevitably shakes, the sealing plate 531 can be transferred to a thinner region, without affecting the penetration and sealing of other channels. For example, the transitional region 5313 may be formed as a depression on one side of the sealing plate 531, or may be formed as depressions on both sides of the sealing plate 531 respectively.

Preferably, the thickness of the transitional region 5313 gradually increases from the periphery of the sealing opening 530 to an inner periphery of the edge region 5312. Such a structural design makes the resistance at the sealing opening 530 smaller when the instrument is inserted and pulled out, so that the instrument can move more smoothly.

In an embodiment, referring to FIG. 9, the sealing plate 531 includes a circular structure. For example, when the instrument channel 50 includes one instrument channel 50, the first portion 5310 includes one first portion 531, and the first portion 5310 may include a fan-shaped structure in the circular structure, for example, the first portion 5310 includes the circular structure. For example, when the instrument channel 50 includes two instrument channels 50, the first portion 5310 includes two first portions 5310, and the first portion 5310 may include a fan-shaped structure in the circular structure, for example, the first part 5310 includes a semicircle of the circular structure. For example, when the instrument channel 50 includes four instrument channels 50, the first portion 5310 includes four first portions 5310, and the first portion 5310 may a fan-shaped structure in the circular structure, for example, the first portion 5310 includes a fan-shaped structure which is one quarter of the circular structure. In the above example, the circle center of the fan-shaped structure is the same as the circle center of the circular structure, in which, "the same" includes exactly the same and substantially the same. In this embodiment, the region of the transitional region 5313 that provides deformation is primarily concentrated in the region of the sealing opening 530 near the peripheral side (i.e. arc) of the circular structure. In an embodiment, the structure of the middle region 5311 is determined corresponding to the structure of the first portion 5310, for example, the middle region 5311 includes a fan-shaped structure.

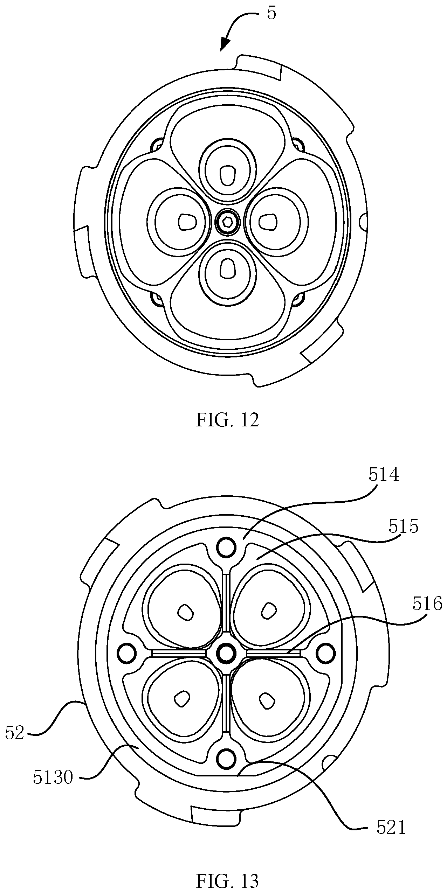

In an embodiment, referring to FIGS. 6 and 7, the sealing plate 531 is disposed at the engaged position between the first body 51 and the second body 52. Also referring to FIGS. 12-14, in the engaged position, the first body 51 is provided with a first boss 513, the second body 52 is provided with a second boss 514, the structure of the first boss 513 and the second boss 514 is matched with the structure of the edge region 5312, the second boss 514 supports the edge region 5312, and the first boss 513 tightly presses the edge region 5312 to the second boss 514. When the sealing plate 531 is pressed between the first boss 513 and the second boss 514, the sealing plate 531 seals the gap between the first boss 513 and the second boss 514. In an embodiment, the threaded hole 71 can be provided on the first boss 513 and the second boss 514, and the first boss 513 and the second boss 514 are locked by the screw 72, so as to further tightly press the sealing plate 531 between the first boss 513 and the second boss 514.

Preferably, the second boss 514 is recessed to form a groove 515, the structure of the groove 515 is matched with the structure of the transitional region 5313, in the condition that the first boss 513 presses the edge region 5312 tightly to the second boss 514, the transitional region 5313 is aligned with the groove 515. The design of the groove 515 can provide sufficient deformation space for the stress deformation of the transitional region 5313, which is beneficial to maintain the sealing.

Preferably, referring to FIGS. 10 and 13, the first boss 513 is provided with a mounting rib 516, the sealing plate 531 is provided with a mounting groove 517 adapted to the mounting rib 516, and the sealing plate 531 is limited by the wedging between the mounting groove 517 and the mounting rib 516 when the sealing plate 531 is mounted on the first boss 513. Further, the second boss 514 presses the sealing plate 531 on the mounting rib 516. In this way, even if the instrument is shaken, the sealing plate 531 is not easily disengaged from the second boss 514, In particular, due to the arrangement of the transitional region 5313 in the sealing plate 531, most of the force generated by the instrument shaking on the sealing opening 530 is transmitted away from the mounting rib 516, and the sealing plate 531 is more difficult to be disengaged from the second boss 514.

In one embodiment, referring to FIG. 10 and FIG. 13, there are four instrument channels 50, the number of sealing openings 530 is four, the different sealing openings 530 are aligned with different instrument channels 50, the sealing plates 531 are pressed between the first body 51 and the second body 52. At the engaged position of the first body 51 and the second body 52, the second body 52 is provided with two orthogonal mounting ribs 516, the four instrument channels 50 are isolated from each other and are respectively located in four quadrants formed by the orthogonal mounting ribs 516, and the sealing plate 531 is provided with two orthogonal mounting grooves 517 matching the mounting ribs 516. The sealing plate 531 is limited by the wedging between the orthogonal mounting grooves 517 and the orthogonal mounting ribs 516 when the sealing plate 531 is mounted on the second body 52.

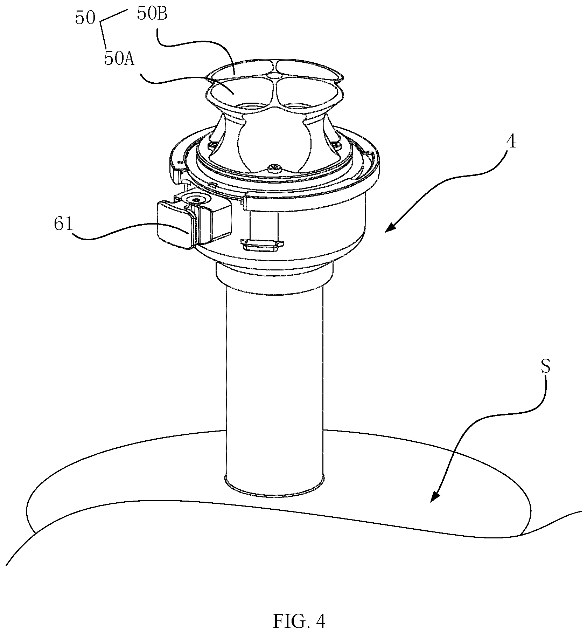

The sizes of the plurality of instrument channels 50 may be equal, partially equal, or unequal. Preferably, referring to FIG. 4, the instrument channel 50 includes two first instrument channels 50A having a first size and two second instrument channels 50B having a second size, the first size being larger than the second size. The two first instrument channels 50A are diagonally arranged, the two second instrument channels 50B are diagonally arranged, and the two first instrument channels 50A and the two second instrument channels 50B are orthogonal. The instrument includes a first instrument having a third size and a second instrument having a fourth size. The third dimension is smaller than the first size and larger than the second size, and the fourth size is smaller than the second size. The first instrument channel 50A allows the first instrument and the second instrument to pass through, and the second instrument channel 50B allows only the second instrument to pass. By way of example, the first instrument includes a surgical instrument whose distal portion is an imaging end instrument, such as an endoscope, and the second instrument includes a surgical instrument whose distal portion is an operating end instrument, such as an electrosurgical tools. In an embodiment, when the sealing device 5 is installed on the guiding cannula 6, the orientation of the first instrument channel 50A is associated with the orientation of the connecting insert 61 of the guiding cannula 6, for example, the orientation of the first instrument channel 50A is the same as the orientation of the connecting insert 61 of the guiding cannula 6, so that the at least one first instrument channel 50A is always located above the second instrument channel 50B, The first instrument installed in the first instrument channel 50A, such as an endoscopic instrument, always on top of the second instrument.

Preferably, referring to FIG. 9, FIG. 13 and FIG. 14, the second body 52 is provided with a first fool-proof structure 521 at the engaged position, a second fool-proof structure 522 is disposed on the periphery of the sealing plate 531, and the first body 51 is provided with a third fool-proof structure 523 at the engaged position, and the second fool-proof structure 522 and the third fool-proof structure 523 are matched with the first fool-proof structure 521. In one embodiment, at the engaged position, the second body 52 has a first boss 513 and a side wall 5130 extending toward the first body 51 along the periphery of the first boss 513, the first boss 513 and the side wall 5130 form an accommodating cavity (not shown), the size of the sealing plate 531 is equal to the size of the accommodating cavity. When the sealing plate 531 is installed on the second body 52, the sealing plate 531 is accommodated in the accommodating cavity and supported by the first boss 513. Further, the first fool-proof structure 521 is disposed on an inner side of the side wall 5130. Exemplarily, the first fool-proof structure 521 includes a convex structure, the second fool-proof structure 522 includes a concave structure, the third fool-proof structure 523 includes a concave structure, the cooperation between the first fool-proof structure 521 and the second fool-proof structure 522 facilitates the positioning between the sealing plate 531 and the second body 52, and the cooperation of the first fool-proof structure 521 and the third fool-proof structure 523 facilitates the positioning between the first body 51 and the second body 52.

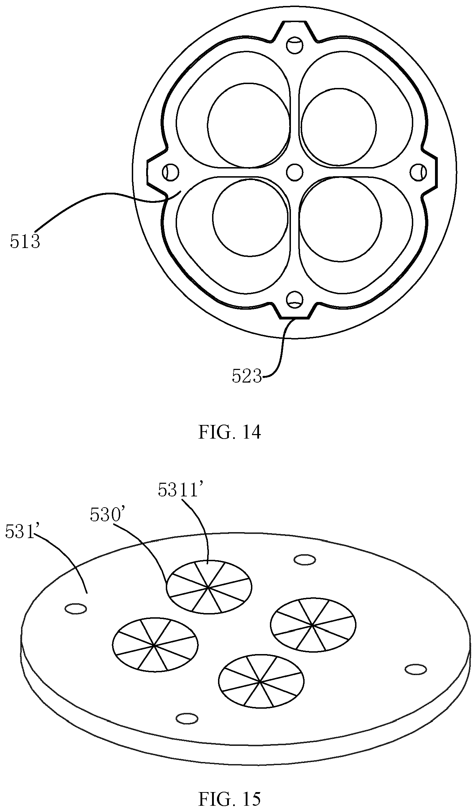

In one embodiment, as shown in FIG. 15, the sealing portion 53 includes another sealing plate 531' having a sealing opening 530' having a diameter smaller than the diameter of the instrument, the diameter of the instrument including the diameter of the connecting rod 31 of the surgical instrument 3. The sealing opening 530' is aligned with the instrument channel 50, and the edge of the sealing opening 530' extends toward the center of the sealing opening 530' to form a plurality of openable flaps 5311'. In a state where the instrument is inserted into the sealing opening 530', the plurality of flaps 5311' are opened; or, in a state where the instrument is not inserted into the sealing opening 530', the plurality of flaps 5311' are closed to form a sealing position. Preferably, the plurality of flaps 5311' include p flaps with a high elastic coefficient to form a sealing position when the instrument is pulled out of the sealing opening 530'. In this embodiment, the sealing door 532 that seals the instrument outlet 501 may be retained or omitted. In this embodiment, the sealing plate 531 may be replaced by the sealing plate 531', or the sealing plate 531' may also be used in combination with the sealing plate 531.

For example, the plurality of flaps 5311' include the same or different arc angles, and the centers of the plurality of flaps 5311' include a circle center of the sealing opening 530'.

Preferably, the sealing portion 53 includes at least two sealing plates 531', the sealing plates 531' are stacked, and multiple layers of the sealing plates 531' can ensure sealing performance.

Preferably, the plurality of flaps 5311' of the adjacent sealing plate 531' are staggered to help improve sealing.

In one embodiment, the sealing portion 53 includes a sealing plate made of a porous elastic material of high polymer, and/or the sealing portion 53 includes a sealing plate made of an aerogel material. In the state that the instrument is inserted into the sealing plate, the sealing plate deforms in the insertion area of the instrument to generate a sealing opening for the instrument to pass through, and the sealing opening abuts against the instrument tightly to form a sealing position; and/or, in the state that the instrument is not inserted into the sealing plate, the sealing plate remains flat or returns to flattening to form a sealing position. In this embodiment, the sealing door 532 that seals the instrument outlet 501 may be retained or omitted. In this embodiment, such a sealing plate may replace the sealing plate 531 and/or the sealing plate 531', or may also be used in combination with the sealing plate 531 and/or the sealing plate 531'.

In one embodiment, referring to FIG. 5, the outer periphery of the second body 52 protrudes outwards to form a block 525, the inner periphery of the top portion of the guiding cannula 6 is provided with a slot 62 for accommodating the block 525. Further, a limiting structure 64 is arranged in the slot 62, and the block 525 is rotatable in the slot 62 and abuts against the limiting mechanism 64 to achieve installation. In an embodiment, when the block 525 abuts against the slot 62, it can be ensured that the orientation of the first instrument channel 50 is associated with the orientation of the connecting insert 61 of the guiding cannula 6, for example, the orientation of the first instrument channel 50 is the same as the orientation of the connecting insert 61 of the guiding cannula 6.

In one embodiment, at least one block 525 is provided on the outer periphery of the sealing device 5, for example, the outer periphery of the second body 52, at least one notch 63 is provided on the inner periphery of the guiding cannula 6, for example, the inner periphery of the top of the guiding cannula 6, the notch 63 is connected to the slot 62, the notch 63 is adapted to the block 525 to allow the block 525 to be inserted into the slot 62 from the corresponding notch 63 along the axial direction of the guiding cannula 6, and then the second body 52 and the guiding cannula 6 are rotated relative to each other, so that the block 525 and the notch 63 are staggered, and then the block 525 is limited in the slot 62 without being disengaged from the notch 63, thereby achieving the connection between the second body 52 and the guiding cannula 6.

Preferably, the block 525 and the notch 63 can be designed with a matching fool-proof structure, so that the block 525 in the second body 52 can only be inserted into the slot 62 corresponding to the specific notch 63 in the guiding cannula 6. Exemplarily, the blocks 525 may be designed in different sizes, and the notches 63 may be designed in different sizes correspondingly. For example, the block 525 includes more than two blocks, the block 525 includes a first block having a first size and a second block having a second size, the number of the notches 63 is not smaller than, for example, equal to, the number of the blocks 525, and the notch 63 includes a first notch having a third size and a second notch having a fourth size, in which, the first size is smaller than the second size, the third size is greater than the first size and smaller than the second size, the fourth size is greater than the second size. Furthermore, only when the first block is aligned with the first notch and the second block is aligned with the second notch, the block can be inserted into the slot.

In an embodiment, referring to FIG. 7, a mounting recess (not shown) is formed on the outer periphery of the second body 52, the mounting recess is sleeved with the annular ring seal 527, the annular ring seal 527 is made of a flexible material, preferably, the annular ring seal 527 is made of an elastic material such as silicone gel. The annular ring seal 527 is configured to achieve a seal between the second body 52 and the guiding cannula 6 when it is connected with the guiding cannula 6.

In the above embodiment, the cross-section of the instrument channel 50 may be circular, and the cross-section of the instrument channel 50 may also be non-circular, which may be determined according to space requirements.

In the above embodiment, the first body 51 and the second body 52 may be split structures, and the first body 51 and the second body 52 may also be of an integrated structure, for example, the first body 51 and the second body 52 may be integrally formed to achieve an integrated structure.

The technical features of the above-described embodiments can be combined in any way. For brevity of the description, all possible combinations of the technical features in the above-described embodiments are not described. However, as long as there is no contradiction between the combinations of these technical features, such combination should be considered as the scope of this specification.

The above-described embodiments have only expressed several embodiments of the present disclosure, which are described in more specific and detailed, but are not therefore to be construed as limiting the scope of the present disclosure. It should be noted that variations and modifications may be made to those of skill in the art without departing from the spirit of the present disclosure, all of which fall within the scope of the present disclosure. Therefore, the scope of protection of the patent of the present application shall be subject to the appended claims.

## Claims

1. A sealing device, comprising:
a first body;
a second body, wherein the second body is connected to the first body to form a through instrument channel for an instrument to pass through; and
a sealing portion, wherein the sealing portion is arranged in the instrument channel, and configured to allow the instrument to pass through, so as to form at least one sealing position in the instrument channel when the instrument is inserted and/or is not inserted into the instrument channel;
wherein
the sealing portion comprises a sealing plate and a sealing door, the sealing plate is provided with a sealing opening, the sealing opening is aligned with the instrument channel, the sealing opening is configured for allowing the instrument to pass through, and in a state that the instrument is inserted into the instrument channel, an inner periphery of the sealing opening abuts against the instrument to form a sealing position; and
the instrument channel comprises an instrument outlet, the instrument outlet is disposed on the second body, and the sealing door is rotatably connected to the second body to seal the instrument outlet in an openable manner.

2. The sealing device of claim 1, wherein the sealing portion comprises an elastic element, the elastic element is connected to the second body and the sealing door, and the elastic element provides an elastic force for pressing the sealing door on the instrument outlet to achieve sealing.

3. The sealing device of claim 1, wherein an included angle between a plane where the instrument outlet is located and a central axis of the first body and/or the second body is 15°-45°; and
the sealing door has an opening and closing angle of 15°-45°.

4. The sealing device of claim 1, wherein the sealing door comprises a rotating shaft, and a height between the sealing plate and the rotating shaft is related to a cross-sectional area of the instrument channel located between the sealing plate and the instrument outlet.

5. The sealing device of claim 4, wherein the height is negatively correlated with the cross-sectional area; and
the height is between 0 mm and 46 mm.

6. The sealing device of claim 1, wherein a protrusion is provided on one side of the sealing door that seals the instrument outlet, and the protrusion is accommodated in the instrument channel when the sealing door is closed in the instrument outlet.

7. The sealing device of claim 6, wherein the protrusion has an arc chamfer,
a mounting slot is formed along a periphery of a base of the protrusion, the mounting slot is sleeved with a sealing ring, and when the sealing door is closed in the instrument outlet, the sealing ring covers a gap between the sealing door and the instrument outlet.

8. The sealing device of claim 1, wherein the sealing door has a first magnetic portion, the instrument outlet has a second magnetic portion that attracts the first magnetic portion, and the sealing position is sealed between the sealing door and the instrument outlet through a magnetic connection between the first magnetic portion and the second magnetic portion.

9. The sealing device of claim 1, wherein the first body is provided with a first channel, the instrument channel comprises the first channel, the first channel comprises a first sub-channel and a second sub-channel that are interconnected, the first sub-channel comprises an instrument inlet and a first middle hole, the second sub-channel comprises the first middle hole and a second middle hole, the instrument inlet, the first middle hole and the second middle hole are successively connected, and a size of the first middle hole is smaller than a size of the instrument inlet.

10. The sealing device of claim 9, wherein the first sub-channel gradually shrinks from the instrument inlet to the first middle hole; and/or the second sub-channel gradually expands from the first middle hole to the second middle hole; and
the second body is provided with a second channel, the instrument channel comprises the second channel, the second channel comprises a third middle hole and an instrument outlet, and the instrument inlet, the first middle hole, the second middle hole, the third middle hole and the instrument outlet are connected in sequence.

11. The sealing device of claim 1, wherein the sealing plate comprises a first portion, the first portion comprises a middle region and an edge region disposed around the middle region, the sealing opening is disposed in the middle region, the middle region comprises a transitional region, the transitional region comprises a region extending from a periphery of the sealing opening to an inner periphery of the edge region, and a thickness of the transitional region is smaller than a thickness of the edge region.

12. The sealing device of claim 11, wherein the thickness of the transitional region increases gradually from the periphery of the sealing opening to the inner periphery of the edge region, and the middle region is fan-shaped.

13. The sealing device of claim 12, wherein the sealing plate is arranged at an engaged position of the first body and the second body, at the engaged position, the first body is provided with a first boss, the second body is provided with a second boss, the structures of the first boss and the second boss are matched with the structure of the edge region, the second boss supports the edge region, and the first boss tightly compacts the edge region on the second boss.

14. The sealing device of claim 13, wherein the second boss is recessed to form a groove, a structure of the groove matches a structure of the transitional region, the first boss compacts the edge region tightly to the second boss, and the transitional region is aligned with the groove; and
the first boss is provided with a mounting rib, the sealing plate is provided with a mounting groove matching the mounting rib, and the sealing plate is limited by wedging between the mounting groove and the mounting rib when the sealing plate is mounted on the second body.

15. The sealing device of claim 13, wherein the instrument channel is one of four instrument channels, the sealing opening is one of four sealing openings, and different sealing openings are aligned with different instrument channels, respectively;
the sealing plate is disposed at an engaged position of the first body and the second body, the first boss is provided with two orthogonal mounting ribs, the sealing plate is provided with two orthogonal mounting grooves matching the mounting ribs, and the sealing plate is limited by wedging between the orthogonal mounting grooves and the orthogonal mounting ribs when the sealing plate is mounted on the second body; and
the four instrument channels comprise two first instrument channels with a first size and two second instrument channels with a second size, the first size is greater than the second size, the two first instrument channels are arranged diagonally, the two second instrument channels are arranged diagonally, and the two first instrument channels and the two second instrument channels are orthogonally arranged.

16. The sealing device of claim 13, wherein the second body is provided with a first fool-proof structure at the engaged position, a periphery of the sealing plate is provided with a second fool-proof structure, the first body is provided with a third fool-proof structure at the engaged position, and the second fool-proof structure and the third fool-proof structure are matched with the first fool-proof structure.

17. The sealing device of claim 1, wherein the sealing portion comprises a sealing plate, the sealing plate has a sealing opening with a diameter smaller than that of the instrument, the sealing opening is aligned with the instrument channel, and an edge of the sealing opening extends towards a center of the sealing opening to form a plurality of openable flaps;
in a state in which the instrument is inserted into the sealing opening, the plurality of flaps are opened; or in a state in which the instrument is not inserted into the sealing opening, the plurality of flaps are closed to form the sealing portion;
the plurality of flaps comprises flaps having a high coefficient of elasticity;
the plurality of flaps comprises fan-shaped flaps having equal or unequal arc angles, and the circle centers of the plurality of flaps comprise a circle center of the sealing opening; and
the sealing portion comprises at least two sealing plates, and the at least two sealing plates are stacked.

18. The sealing device of claim 1, wherein the sealing portion comprises a sealing plate made of a porous elastic material of high polymer, and/or the sealing portion comprises a sealing plate made of an aerogel material;
in a state where the instrument is inserted into the sealing plate, the sealing plate deforms in the insertion area of the instrument to generate a sealing opening for the instrument to pass through, and the sealing opening abuts against the instrument to form the sealing position in the state that the instrument is inserted into the sealing plate; and/or,
in a state where the instrument is not inserted into the sealing plate, the sealing plate remains flat or returns to flat to form the sealing position.

19. A sealing device, comprising:
a first body;
a second body, wherein the second body is connected to the first body to form a through instrument channel for an instrument to pass through; and
a sealing portion, wherein the sealing portion is arranged in the instrument channel, and configured to allow the instrument to pass through, so as to form at least one sealing position in the instrument channel when the instrument is inserted and/or is not inserted into the instrument channel.

20. The sealing device of claim 19, wherein the sealing portion comprises a sealing plate, the sealing plate is provided with a sealing opening, the sealing opening is aligned with the instrument channel, the sealing opening is configured for allowing the instrument to pass through, and in a state that the instrument is inserted into the instrument channel, an inner periphery of the sealing opening abuts against the instrument to form a sealing position.

21. The sealing device of claim 19, wherein the sealing portion comprises a sealing door, the instrument channel comprises an instrument outlet, the instrument outlet is disposed on the second body, and the sealing door is rotatably connected to the second body to seal the instrument outlet in an openable manner.

22. The sealing device of claim 21, wherein the sealing portion comprises an elastic element, the elastic element is connected to the second body and the sealing door, and the elastic element provides an elastic force for pressing the sealing door on the instrument outlet to achieve sealing.

23. The sealing device of claim 21, wherein an included angle between a plane where the instrument outlet is located and a central axis of the first body and/or the second body is 15°-45°, and the sealing door has an opening and closing angle of 15°-45°.

24. The sealing device of claim 21, wherein the sealing door comprises a rotating shaft, and a height between the sealing plate and the rotating shaft is related to a cross-sectional area of the instrument channel located between the sealing plate and the instrument outlet.

25. The sealing device of claim 24, wherein the height is negatively correlated with the cross-sectional area, and the height is between 0 mm and 46 mm.

26. The sealing device of claim 21, wherein a protrusion is provided on one side of the sealing door that seals the instrument outlet, and the protrusion is accommodated in the instrument channel when the sealing door is closed in the instrument outlet.

27. The sealing device of claim 26, wherein the protrusion has an arc chamfer, a mounting slot is formed along a periphery of a base of the protrusion, the mounting slot is sleeved with a sealing ring, and when the sealing door is closed in the instrument outlet, the sealing ring covers a gap between the sealing door and the instrument outlet.

28. The sealing device of claim 21, wherein the sealing door has a first magnetic portion, the instrument outlet has a second magnetic portion that attracts the first magnetic portion, and the sealing position is sealed between the sealing door and the instrument outlet through a magnetic connection between the first magnetic portion and the second magnetic portion.

29. The sealing device of claim 19, wherein the first body is provided with a first channel, the instrument channel comprises the first channel, the first channel comprises a first sub-channel and a second sub-channel that are interconnected, the first sub-channel comprises an instrument inlet and a first middle hole, the second sub-channel comprises the first middle hole and a second middle hole, the instrument inlet, the first middle hole and the second middle hole are successively connected, and a size of the first middle hole is smaller than a size of the instrument inlet.

30. The sealing device of claim 29, wherein the first sub-channel gradually shrinks from the instrument inlet to the first middle hole; and/or the second sub-channel gradually expands from the first middle hole to the second middle hole; and
the second body is provided with a second channel, the instrument channel comprises the second channel, the second channel comprises a third middle hole and an instrument outlet, and the instrument inlet, the first middle hole, the second middle hole, the third middle hole and the instrument outlet are connected in sequence.

31. The sealing device of claim 20, wherein the sealing plate comprises a first portion, the first portion comprises a middle region and an edge region disposed around the middle region, the sealing opening is disposed in the middle region, the middle region comprises a transitional region, the transitional region comprises a region extending from a periphery of the sealing opening to an inner periphery of the edge region, and a thickness of the transitional region is smaller than a thickness of the edge region.

32. The sealing device of claim 31, wherein the thickness of the transitional region increases gradually from the periphery of the sealing opening to the inner periphery of the edge region, and the middle region is fan-shaped.

33. The sealing device of claim 32, wherein the sealing plate is arranged at an engaged position of the first body and the second body, at the engaged position, the first body is provided with a first boss, the second body is provided with a second boss, the structures of the first boss and the second boss are matched with the structure of the edge region, the second boss supports the edge region, and the first boss tightly compacts the edge region on the second boss.

34. The sealing device of claim 33, wherein the second boss is recessed to form a groove, a structure of the groove matches a structure of the transitional region, the first boss compacts the edge region tightly to the second boss, and the transitional region is aligned with the groove; and
the first boss is provided with a mounting rib, the sealing plate is provided with a mounting groove matching the mounting rib, and the sealing plate is limited by wedging between the mounting groove and the mounting rib when the sealing plate is mounted on the second body.

35. The sealing device of claim 33, wherein the instrument channel is one of four instrument channels, the sealing opening is one of four sealing openings, and different sealing openings are aligned with different instrument channels, respectively;
the sealing plate is disposed at an engaged position of the first body and the second body, the first boss is provided with two orthogonal mounting ribs, the sealing plate is provided with two orthogonal mounting grooves matching the mounting ribs, and the sealing plate is limited by wedging between the orthogonal mounting grooves and the orthogonal mounting ribs when the sealing plate is mounted on the second body; and
the four instrument channels comprise two first instrument channels with a first size and two second instrument channels with a second size, the first size is greater than the second size, the two first instrument channels are arranged diagonally, the two second instrument channels are arranged diagonally, and the two first instrument channels and the two second instrument channels are orthogonally arranged.

36. The sealing device of claim 33, wherein the second body is provided with a first fool-proof structure at the engaged position, a periphery of the sealing plate is provided with a second fool-proof structure, the first body is provided with a third fool-proof structure at the engaged position, and the second fool-proof structure and the third fool-proof structure are matched with the first fool-proof structure.

37. The sealing device of claim 19, wherein the sealing portion comprises a sealing plate, the sealing plate has a sealing opening with a diameter smaller than that of the instrument, the sealing opening is aligned with the instrument channel, and an edge of the sealing opening extends towards a center of the sealing opening to form a plurality of openable flaps;
in a state in which the instrument is inserted into the sealing opening, the plurality of flaps are opened; or in a state in which the instrument is not inserted into the sealing opening, the plurality of flaps are closed to form the sealing portion;
the plurality of flaps comprises flaps having a high coefficient of elasticity;
the plurality of flaps comprises fan-shaped flaps having equal or unequal arc angles, and the circle centers of the plurality of flaps comprise a circle center of the sealing opening; and
the sealing portion comprises at least two sealing plates, and the at least two sealing plates are stacked.

38. The sealing device of claim 19, wherein the sealing portion comprises a sealing plate made of a porous elastic material of high polymer, and/or the sealing portion comprises a sealing plate made of an aerogel material;
in a state where the instrument is inserted into the sealing plate, the sealing plate deforms in the insertion area of the instrument to generate a sealing opening for the instrument to pass through, and the sealing opening abuts against the instrument to form the sealing position in the state that the instrument is inserted into the sealing plate; and/or,
in a state where the instrument is not inserted into the sealing plate, the sealing plate remains flat or returns to flat to form the sealing position.

39. A puncture device, comprising:
a guiding cannula, wherein an inner periphery of the guiding cannula is provided with a notch and a slot that communicate with each other; and
a sealing device, wherein the sealing device comprising:
a first body;
a second body, wherein an outer periphery of the second body is provided with a block, the second body is connected to the first body to form a through instrument channel for an instrument to pass through; and
a sealing portion, wherein the sealing portion is arranged in the instrument channel, and configured to allow the instrument to pass through, so as to form at least one sealing position in the instrument channel when the instrument is inserted and/or is not inserted into the instrument channel;
wherein
the block is inserted from the notch and accommodated in the slot to realize connection between the guiding cannula and the sealing device.
